# EUROPEAN PATENT SPECIFICATION

(11) **EP 1 159 978 B1**
(45) Date of publication and mention of the grant of the patent: **10.11.2004**
(21) Application number: 01500086.2
(22) Date of filing: 29.03.2001
(51) Int. Cl.: A61M 5/28, A61M 5/31

(54) **Discoid and elastic valve to produce a hermetic closure inside pre-filled syringes**
Diskusförmiges und elastisches Ventil zum Erzeugen eines hermetischen Verschlusses innerhalb von vorgefüllten Spritzen
Soupape discoidale et élastique pour obtenir une fermeture hermétique à l'intérieur de seringues préremplies

(30) Priority: 05.04.2000 AR 0101565
(43) Date of publication of application: 05.12.2001
(73) Proprietor: Szapiro Melamed, Jaime Luis, Buenos Aires (AR); Moreno Bonino, Saul, Buenos Aires (AR); Szames Yungelson, Leonardo, Buenos Aires (AR)
(72) Inventor: Szapiro Melamed, Jaime Luis, Buenos Aires (AR)
(74) Representative: Toro Gordillo, Ignacio Maria

(56) References cited:
- EP-A- 0 974 373
- DE-A- 2 455 631
- US-A- 3 685 514
- US-A- 4 919 655

## Description

This patent has as a main subject a syringe comprising a DISCOID AND ELASTIC VALVE TO PRODUCE AN HERMETIC CLOSURE INSIDE PRE-FILLED SYRINGES, keeping the liquid content to be injected totally isolated, in order to avoid the liquid to get in contact with the needle or the external air.

Specifically, this patent discloses a DISCOID AND ELASTIC VALVE especially designed to block up the neck of the syringe from inside, in order to close the communication between the syringe's internal main body, where the liquid is, and the needle which is connected in the syringe neck through its plugging cone.

As it was said, the hermetic closure valve is specially designed to operate in pre-filled syringes.

The characteristic feature of all these pre-filled syringes is that they have a cylindrical and hollow main body, with a distal end where a communication neck with the injection needle is defined, while inside a coaxially set and manually movable plunger is included, which extends itself outwards from the proximal end of the main body which is totally open. All of these known syringes have an inner chamber formed inside the main body, constituting the temporary location of the liquid to be injected. This inner chamber is determined by the cylindrical wall of the main body, the above mentioned distal neck where the needle and the active head of the manually movable plunger fit, this active head tightly rests on the internal face of the cylindrical wall of the main body.

In this kind of pre-filled syringes, needles are located inside a protecting sheath that, at the same time closes and couples with the syringe, keeping it quiet and isolated, has coupling resources to the syringe.

It is observed then that the referred coupling resources, the ones of the needle with respect to the main body of the syringe, as well as its protecting sheath, are the ones that properly ensure the permanent isolation of the liquid contained, until the injection. In order to achieve this, the same coupling set in the back of the needle, forms a hermetic closure against the inner wall of the sheath at the mentioned zone.

### PRIOR ART

These known syringes have given excellent results in practice and approved different experiments that guarantee water tightness in zones of location and circulation of liquid, as well as security in the safe use for the patient and operator. Nevertheless, other devices have appeared which, besides the above-mentioned closing resources, incorporate other plugs specially created to improve even more the referred isolation of the liquid.

In this sense, Argentine patent N.250.277 is mentioned as an antecedent, which supports a very special valve plug whose function consists in keeping the liquid isolated inside of syringe body avoiding the contact with the needle until the injection is performed. Consequently, during the placement of the needle coupling it to the plugging cone or syringe neck and withdrawal of the protecting sheath to perform the injection, the liquid is kept isolated inside the main body of the syringe, ensuring in this way the impossibility that these coupling and uncoupling actions may cause some unwanted loss or spilling.

A plug is specially designed to be placed in the referring neck occluding the same from the outlet mouth. It consists of a substantially cylindrical solid body which has a section with a conical end, while in the other end the head of the plug is defined, which has a major transversal section than the rest and set to rest on the outlet mouth of the neck. This plug outstands because besides the mentioned conical end of the cylindrical body, it has at least two faceted faces that extend up to the adjacencies of the head.

It is important to underline that the plug supported by the mentioned patent N.250.277 is specially designed for the mouth of the syringe neck that faces the injection needle. Due to its special shape the same releases the liquid outlet only when hydraulic pressure is produced from the plunger towards the syringe. This pressure partially removes the plug and the liquid flows.

EP-A-0 974 373 discloses a syringe with a valve inside the syringe barrel according to the preamble of claim 1. US-A-3 685 514 and DE 24 55 631 show elastic single body valves in syringe applications.

### NOVELTY OF THE INVENTION AND MAIN PURPOSE

The present invention consists in a syringe according to claim 1.

The discoid valve is also specially designed to isolate the liquid contained in a pre-filled syringe, avoiding its contact with the needle and it clearly differs from the cited antecedent due to the fact that it works operatively in a different way.

In fact, the valve is located inside the main body to perform the closing action in the origin of the communication duct that defines the neck of the syringe. The valve has a coaxial stub that is the one that fits in the inner part of the syringe neck closing it and forming a hermetic chamber that contains the liquid to be injected, whose limits are determined by the internal walls of the syringe body and the active head of the plunger.

This new arrangement combined with the elastic condition is the one that ensures the impossibility of unwanted losses of liquid, with the peculiarity that, when being inside the syringe body, every manipulation that may be performed during the coupling and uncoupling of the injection needle, as well as its respective protection sheath, may never influence on that hermetic closure.

Likewise, if the plunger is accidentally pushed in an injection action, the produced closure will be greater.

The new functional conception incorporated with the invented valve consists in that it is necessary that the plunger move in an opposite direction of the one of the injection in order to provide a free flowing of the liquid towards the needle. In such case the mentioned stub is removed from its location and the valve withdraws itself releasing the passage of liquid.

This action, which is not possible to perform with other plugs of this kind, is very important for the syringe operator since, if the needle is inserted in a vein, the action indicated for the release of the closing stub, allows to prove if the needle is inserted correctly. As it is known, if the needle is inserted in a vein, the sucking action indicated, causes the entrance of blood in the syringe which is nothing but a desirable and necessary evidence every time one tries to inject a liquid, since the injection could be intravenous or not, it is necessary to ensure that the destiny of the liquid is the one wanted.

Another characteristic of the valve is that the cylindrical stub has a cap-shaped higher base.

Likewise, it is underlined that the cylindrical stub projects from the center of the flat and elastic base.

On the other hand, the holes neatly distributed are preferably circular.

The perimetrical ring is circumferential and thicker than the elastic base, and it is preferably a rigid ring.

### BRIEF DESCRIPTION OF FIGURES

In order to specify the advantages briefly commented, to which users and specialist may add many more and in order to make easy the understanding of the constructive, constituent and functional characteristics of the invented discoid and elastic valve, it is described next
A preferred performing example, which is illustrated, in outline and without a determine scale, in the enclosed sheets, with the explanation that, for being precisely an example, it is not suitable to assign the same a limitative or exclusive criteria of the protection extent of this letters patent, but it simply corresponds a merely explicative and illustrative intention of the basis conception in which the same is based.

Figure N.1 is a top view that represents a discoid valve like the one supported in this document.

Figure N. 2 is a cross-section of the same discoid valve represented en the previous figure.

Figure N. 3 is a vertical section of a syringe including, inside its main body, a discoid valve, according to this invention, adopting the closing position.

Figure N.4 is a vertical section of a syringe that includes the invented discoid valve, in this case in an open disposition to allow the passage of liquid.

Figure N. 5 is also a vertical section that shows the disposition of the plunger when it has moved in the direction of the injection, evacuating much of the liquid contained in the syringe.

Figure N. 6 is also a vertical section where the disposition of the invented valve after injecting all the liquid is shown.

It should be explained that, in every figure, same reference numbers correspond to same or equivalent parts or constitutive elements of the group, according to the example chosen for the present explanation.

### DETAILED DESCRIPTION OF A PERFORMING EXAMPLE

As it may be appreciated in Figures N. 1 and 2, the discoid and elastic valve to produce an hermetic closure inside pre-filled syringes to which this patent refers, is a single body that comprises a central stub -1- with a cylindrical shape and a cap-shaped higher base that projects from a flat and elastic base -2- with a circular form in which a series of properly distributed holes are defined -3-. This elastic base -2- is limited by a circumferential ring -4- that acts as perimetric border of the valve.

If we now observe figures N. 3 to 6 it is possible to appreciate that this discoid valve is specially designed to operate in pre-filled syringes such as the ones defined, that comprise a syringe body -13- with a cylindrical shape, inside of which the plunger is placed -5- that is manually movable and has its active head -6-which is the one that determines the bottom of a chamber of variable volume -7- that is where the liquid product to be supplied is located.

The above mentioned internal chamber-7- is limited bay the cylindrical wall of the main body -13-, the cited active head -6- and a front base where the neck originates -8- that together with the wall -9- define the "plugging cone" or coupling means for the injection needle.

In these same figures, with the reference -10- the mentioned needle that, through the middle -11-couples with the syringe assembling in the mentioned "plugging cone" -8/9- is represented. The sheath -12 - that also couples the mentioned plugging cone -8/9-, protects this whole group.

Thus the group is constituted, and as figure N. 3 shows, the invented valve is suitable to be placed on the base -14- of the syringe body in a way that the stub -1- clearly closes the communication passage towards the needle -10-. In this way a hermetic chamber is formed -7- where the liquid to be injected is located.

It is underlined that the way the valve of this invention is placed, it is impossible to have spilling of the contained liquid, also during the movements and manipulations that occur during the placement of the needle -10- and the placement or removal of its protective sheath-12-. In fact, the valve prevents any possibility of liquid loss.

Observing now Figure N. 4, it can be appreciated that, once the protective sheath is removed -12- and with the needle in place-11- in use position, the operator can move the plunger in the direction indicated with the reference -F-. The effect produced consists in the deformation of the elastic base -2- until the stub -1-releases the passage (through the inside of the neck -8-) towards the injection needle. The liquid located in chamber -7- goes through the mentioned holes -3-and in this way is led through the inside of the neck -8- towards the needle.

The valve always remains in the same position because its circumferential ring fits tightly in the internal face of the cylindrical wall of the syringe body, with a higher adjusting pressure than the one required to bend the elasticity this base has -2-and deforms itself in the direction and sense -F- in order to remove the stub -1-from its closing position.

As Figure N.4 shows, if the needle -12 is inserted in a vein, such releasing action by elastic deformation of the base -2- will cause the entrance of blood in the syringe, which is a necessary and common evidence every time one tries to inject a liquid, since the injection, could be intravenous or not, is suitable to ensure that the destiny of the liquid is the one wanted.

If we observe now Figures N. 5 and 6 it may be appreciated that with the movement of the plunger in the direction -F1- it is possible to remove the whole liquid contained inside the chamber-7- since the same flows through the holes -3-until the valve adopts the initial position, that is to say, with the plug -1- closing the neck passage -8-.

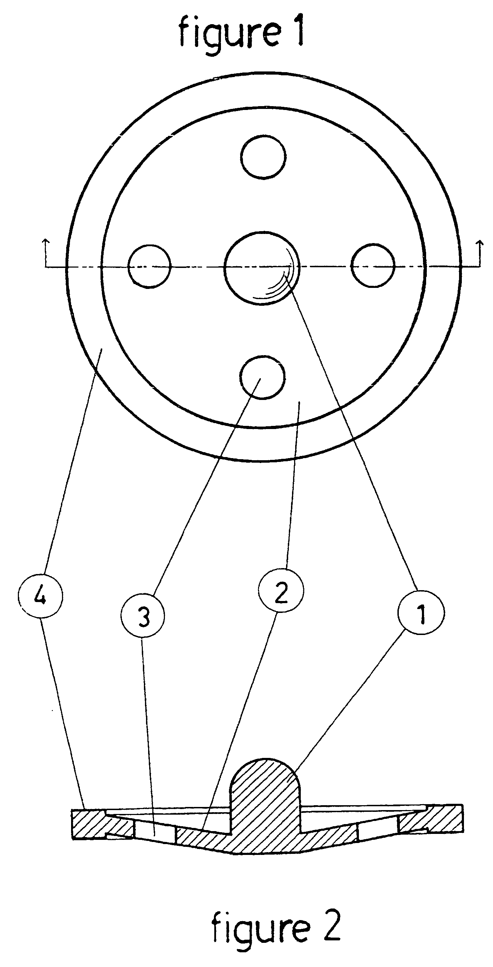

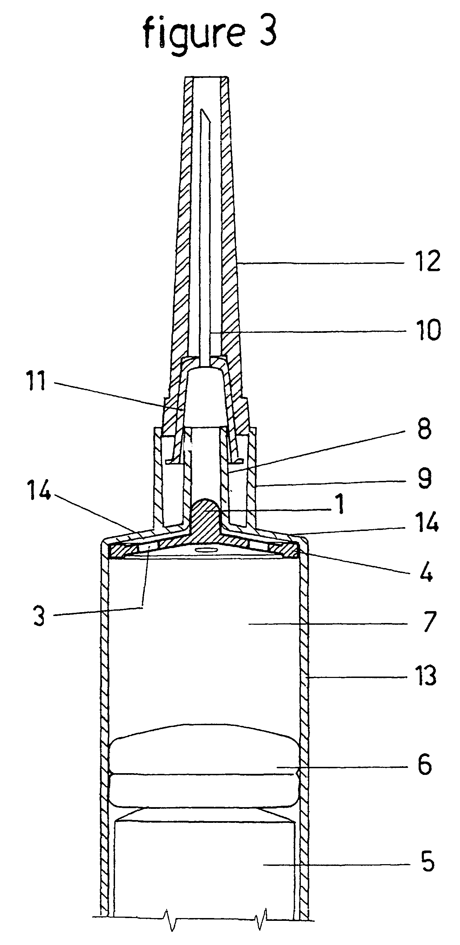

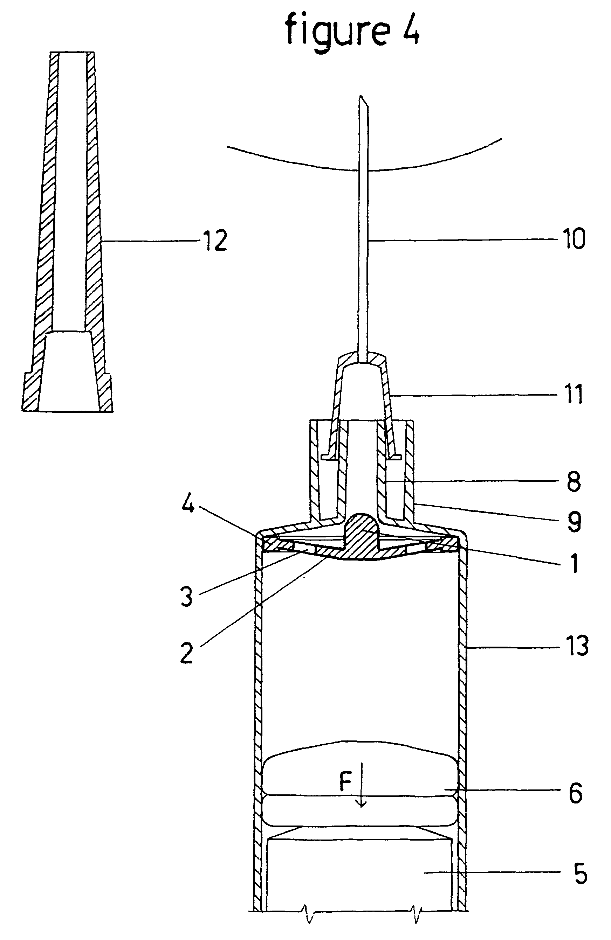

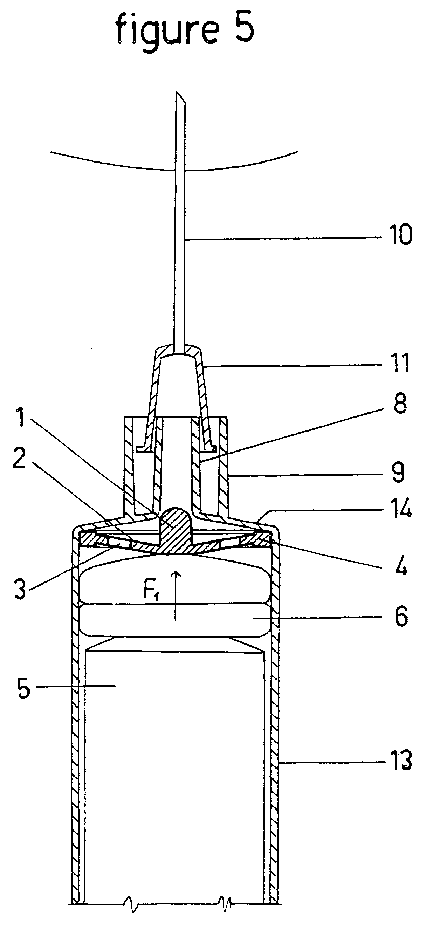

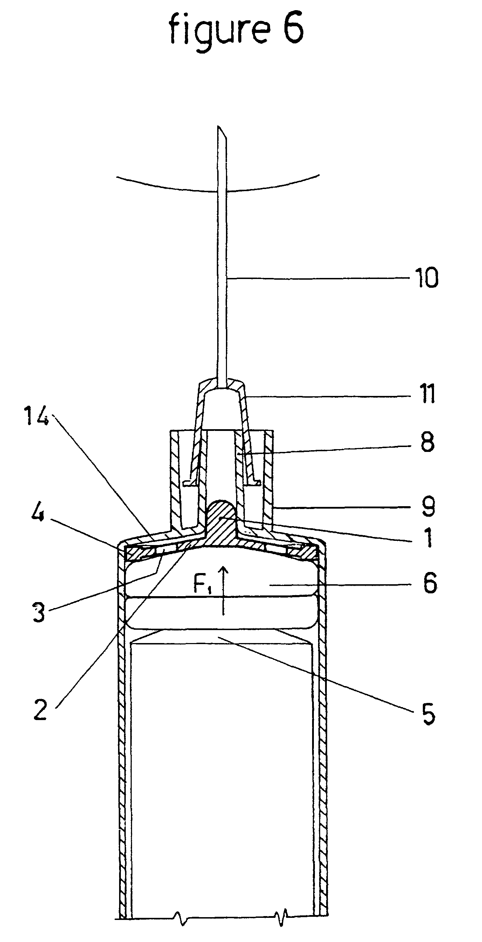

## Claims

1. Syringe comprising a barrel (13) having a proximal and a distal end, and a **DISCOID AND ELASTIC VALVE TO PRODUCE A HERMETIC CLOSURE INSIDE PRE-FILLED SYRINGES,** the valve being placed inside the syringe barrel and closing the communication passage at the distal end of the barrel (13) towards an injection needle (10) that is fitted on a neck (8) of the syringe barrel, the valve being **characterised in** consisting in a single body that comprises a cylindrical stub (1) that projects perpendicularly from a flat and elastic base (2) with a circular shape, the base including a series of holes (3) through the base, which are neatly distributed, the base being limited by a perimetric ring (4) that fits tightly on the internal face of the cylindrical wall of the syringe barrel (13), the cylindrical stub (1) getting removed from the communication passage to open the valve upon drawing a plunger (5) having a piston (6) and being inserted in the syringe barrel towards the proximal end of the barrel, the valve remaining in the opened position when subsequently pushing the plunger (5) towards the distal end of the barrel to express the content of the syringe barrel through the needle (10).

2. Syringe according to what is claimed in claim 1, wherein the cylindrical stub (1) has a cap-shaped higher base.

3. Syringe according to what is claimed in claim 1, wherein the holes neatly distributed are circular.

4. Syringe according to what is claimed in claim 1, wherein the perimetric ring is circumferential.

5. Syringe according to what is claimed in claim 1, wherein the perimetric ring is thicker than the elastic base.

6. Syringe according to what is claimed in claim 1, wherein the perimetric ring is rigid.

## Patentansprüche

1. Spritze bestehend aus einem Zylinder (13) welcher ein proximales und ein distales Ende hat, und ein SCHEIBENFÖRMIGES UND ELASTISCHES VENTIL, UM EINEN DICHTEN VERSCHLUSS IM INNEREN VON VORGEFÜLLTEN SPRITZEN ZU ERZEUGEN, wobei das Ventil sich im Inneren des Spritzenzylinders befindet und den Verbindungsgang am distalen Ende des Zylinders (13) in der Richtung einer Spritzennadel (10), die auf einen Hals (8) des Spritzenzylinders sitzt, schliesst, wobei das Ventil **dadurch gekennzeichnet ist, dass** es aus einem einzigen Körper besteht, welcher einen zylindrischen Stumpf (1) enthält, der sich senkrecht aus einer flachen und elastischen Basis (2) mit einer kreisförmigen Form erstreckt, wobei die Basis mehrere Löcher (3) durchgehend durch die Basis einschliesst, die ordenlich verteilt sind, wobei die Basis durch einen Umfangsring (4) begrenzt ist, welcher dicht auf die innere Seite der zylindrischen Wand des Spritzenzylinders (13) sitzt, wobei der zylindrische Stumpf (1) von dem Verbindungsgang entfernt wird, um das Ventil zu öffnen (nachdem ein Kolben (5) der einen Piston (6) hat, angezogen wird), und in dem Spritzenzylinder zum proximalen Ende des Zylinders hin eingeführt wird, wobei das Ventil in der offenen Position bleibt, nachdem der Kolben (5) zum distalen Ende des Zylinders hin bewegt wird, um den Inhalt des Spritzenzylinders durch die Nadel (10) auszustossen.

2. Spritze gemäss Anspruch 1, wobei der zylindrische Stumpf (1) eine höhere Kappenförmige Basis hat.

3. Spritze gemäss Anspruch 1, wobei die ordentlich verteilte Löcher kreisrund sind.

4. Spritze gemäss Anspruch 1, wobei der Umfangsring kreisförmig ist.

5. Spritze gemäss Anspruch 1, wobei der Umfangsring dicker als die elastische Basis ist.

6. Spritze gemäss Anspruch 1, wobei der Umfangsring steif ist.

## Revendications

1. Seringue comprenant un canon (13) à terminaison proximale et distale, et une valve, ou clapet, discoïde (destiné à produire une fermeture hermétique dans une seringue déjà remplie), valve, ou clapet, située dans le canon susvisé.
A l'extrémité distale du canon (13), cette valve, ou clapet, située à l'intérieur du canon ferme la communication vers l'aiguille ci ajustée dans le col correspondant. Dans le canon susvisé, ladite valve, ou clapet, consiste en un seul élément que comprend un goujon cylindrique (1) qui se projette perpendiculairement depuis une base plane et élastique (2), de forme circulaire. Cette base comprend une série d'orifices (3) nettement distribués, cette base étant limitée par un anneau (4) couvrant le périmètre, anneau qui s'ajuste étroitement sur la face intérieure de la paroi cylindrique du canon (13). Le goujon cylindrique (1) une fois ôté du passage de communication pour ouvrir la valve, ou clapet, après que le piston (6) ait été mû, étant celui-ci inséré dans le canon (vers l'extrémité proximale de celui-ci), fait que la valve, ou clapet, demeurât en position ouverte lorsqu'en conséquence, le piston est poussé vers l'extrémité distale du canon, pour exprimer le contenu du canon par l'aiguille (10).

2. Seringue conformément à la revendication N° 1, dans laquelle le goujon cylindrique (1) possède une base supérieure en forme de calotte.

3. Seringue conformément à la revendication N° 1 où les orifices nettement distribués sont de forme circulaire.

4. Seringue conformément à la revendication N° 1, dans laquelle l'anneau qui s'ajuste au périmètre, couvre la circonférence.

5. Seringue conformément à la revendication N° 1 où ledit anneau qui s'ajuste au périmètre est plus épais que la base élastique.

6. Seringue conformément à la revendication N° 1, ou ledit anneau qui s'ajuste au périmètre, est rigide.
